(19) Europäisches Patentamt
European Patent Office
Office européen des brevets

(11) Numéro de publication: **0 432 209 B1**

(12) **FASCICULE DE BREVET EUROPEEN**

(45) Date de publication de fascicule du brevet: **02.02.94**

(51) Int. Cl.⁵: **C07D 207/34**, C07D 471/04, C07D 405/06, A61K 31/40, A61K 31/47

(21) Numéro de dépôt: **89910118.2**

(22) Date de dépôt: **04.09.89**

(86) Numéro de dépôt internationale : **PCT/FR89/00442**

(87) Numéro de publication internationale : **WO 90/02733 (22.03.90 90/07)**

(54) **COMPOSES PHENYLPYRROLIOUES UTILES EN TANT OUE MEDICAMENTS, LEUR PREPARATION ET LEUR APPLICATION.**

(30) Priorité: **05.09.88 FR 8811592**

(43) Date de publication de la demande: **19.06.91 Bulletin 91/25**

(45) Mention de la délivrance du brevet: **02.02.94 Bulletin 94/05**

(84) Etats contractants désignés: **AT BE CH DE FR GB IT LI LU NL SE**

(56) Documents cités: **DE-A- 1 545 860**

**CHEMICAL ABSTRACTS, vol. 79, 1973, Columbus, Ohio, US; G.C. PORRETTA et al.: "Compounds with antiblastic activity. III. Synthesis and cytostatic and cytotoxic activity of arylpyrrolecarboxylic acid amides", page 409, résumé no. 91870d**

(73) Titulaire: **CENTRE NATIONAL DE LA RECHERCHE SCIENTIFIOUE
15, quai Anatole France
F-75007 Paris(FR)**

(72) Inventeur: **THAL, Claude
15 ter, rue des clos St Marcel
F-92330 Sceaux(FR)**
Inventeur: **BOYE, Olivier
40, rue des Bordes
F-91450 Etiolles(FR)**
Inventeur: **GUENARD, Daniel
19, rue d'Arcueil
F-92120 Montrouge(FR)**
Inventeur: **POTIER, Pierre
14, avenue de Breteuil
F-75007 Paris(FR)**

(74) Mandataire: **Warcoin, Jacques et al
Cabinet Régimbeau
26, avenue Kléber
F-75116 Paris (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne de nouveaux composés de phénylpyrrole, leur préparation et leur application à titre de principe actif, en particulier en tant que médicament à activité antimitotique.

Le but de la présente invention est de proposer de nouveaux composés utiles dans la lutte contre les cellules cancéreuses, révélés dans un premier temps par leur réactivité vis-à-vis de la tubuline, puis par la mise en évidence de leurs propriétés antimitotiques.

La tubuline est une protéine cellulaire de poids moléculaire de l'ordre de 110 000 à 120 000 daltons, constituée de deux sous-unités $\alpha$ et $\beta$ étroitement associées. Elle constitue un élément de base dont l'assemblage sous forme hélicoïdale permet la construction de structures macromoléculaires complexes communément appelées microtubules. Ceux-ci sont rencontrés dans pratiquement toutes les cellules eucaryotes et servent à la formation de nombreuses architectures cytoplasmiques : fuseau mitotique, centrioles, flagelles, axonèmes, neurotubules... Les microtubules ont ainsi des rôles fondamentaux, non encore tous répertoriés, dans la vie cellulaire (division, motilité, transport, croissance...). L'assemblage de la tubuline est un mécanisme dynamique, réversible et faisant l'objet d'une régulation pour l'instant non élucidée.

Après extraction de la protéine (à partir de la cervelle de porc), on peut suivre in vitro ses comportements d'assemblage et de désassemblage sous l'effet de la variation de différents paramètres physico-chimiques :

- polymérisation sous forme de microtubules à la suite d'une élévation de température à 37 °C ; favorisée par la présence de GTP, de polycations, de glycérol...
- dépolymérisation provoquée par une température basse (4 °C) et favorisée par l'ion $Ca^{2+}$, un excès GTP...

Un certain nombre de substances naturelles sont capables de se fixer sur des sites récepteurs spécifiques de la tubuline. Elles inhibent sa polymérisation (colchicine, vinblastine, vincristine, podophyllotoxine...), sa dépolymérisation (taxol, rhazinilame) et peuvent provoquer sa spiralisation (vinblastine).

La présente invention s'est orientée vers des substances présentant un caractère biarylique, capables d'inter-réagir avec la tubuline et donc présentant une activité en tant que poison du fuseau mitotique. En ce sens, il a été synthétisé des composés à squelette phénylpyrrole.

Les composés, objet de la présente invention, répondent à la formule générale I

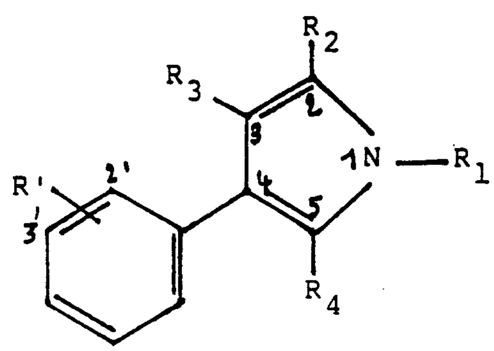

(I)

dans laquelle

- $R_1$, $R_2$ et $R_3$ indépendamment l'un de l'autre sont représentés par :
  (1) - H
  (2) - alcoyle $C_{1-6}$,
- R' situé en position 2' ou 3' est représenté par :
  (1) - H
  (2) - $NO_2$
  (3) -

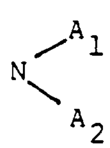

dans lequel $A_1$ et $A_2$ indépendamment l'un de l'autre représentent :

(a) - H

(b) - alcoyle $C_{1-4}$,

(c) - alcoylène en $C_{1-6}$ ou alcénylène en $C_{1-6}$ qui se cyclise sur l'atome de C en position 5 du cycle pyrrole,

(d) -

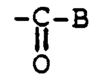

dans lequel B représente :

- phénylalcoxy $C_{1-4}$,
- N-benzyloxycarbonylaminoalcoyle $C_{1-4}$,
- alcoylène en $C_{1-6}$ ou alcénylène en $C_{1-6}$ qui se cyclise sur l'atome de C en position 5 du cycle pyrrole.

- $R_4$ est représenté par :

(1) - hétérocycloalcoyl-(alcoyle $C_{1-4}$) contenant 5 ou 6 chaînons,

(2) -

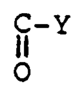

dans lequel Y représente :

(a) - alcoxy $C_{1-4}$

(b) - NN'-cycloalcoyl-(uréido) contenant 5 à 6 chaînons

(c) - amino cyclisable en position R'. Composés 3-phenylpyrroliques et leur utilisation comme agents microleicides sont décrits dans DE-A-1 545 860.

La présente invention se rapporte également à la préparation des composés représentés par la formule générale I. Ce procédé de préparation est caractérisé en ce que l'on fait réagir un nitrostyrène de formule II.

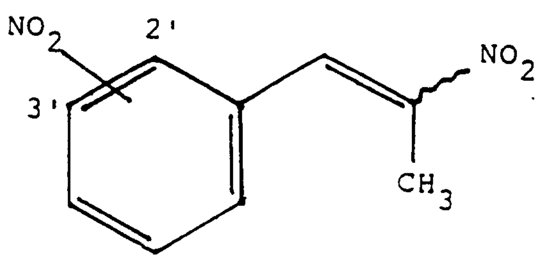

(II)

dans laquelle :
le groupe $NO_2$ est situé en position 2' ou 3', avec un isocyanoacétate d'alcoyle de formule III

$R\text{-}O\text{-}CO\text{-}CH_2\text{-}NC$ (III)

dans laquelle :
R est un groupement éthyle ou méthyle.

La réaction est réalisée selon la méthode de Barton et Zard permettant la formation de pyrroles à partir de nitroalcènes aliphatiques ou aromatiques, dans un mélange THF/tertiobutanol absolu (2/1) à une température de 40-50 °C. Le point de départ de la réaction est l'addition de type Michaël d'un α isocyanoacétate sur le nitrostyrène en présence d'une base, dans ce cas, le D.B.U.

Le nitrostyrène utilisé, est préliminairement préparé par condensation de nitroéthane sur le nitrobenzaldéhyde correspondant selon une réaction de Knoevenagel.

A partir des nitrostyrènes, on accède donc de manière directe ou par une transformation fonctionnelle simple, aux phénylpyrroles, objet de la présente invention.

Ainsi, dans le cas où le composé I' est obtenu

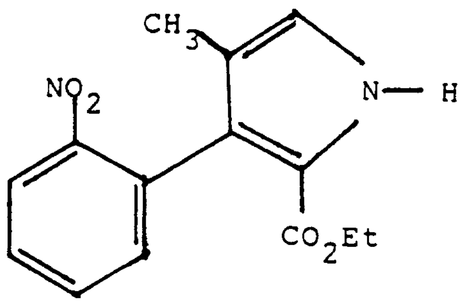

I'

il est transformé en d'autres composés représentés par la formule générale I selon l'une ou plusieurs des transformations suivantes :

(1) - réduction simultanée du groupe nitro du cycle aromatique et de la fonction ester du cycle pyrrole et dans ce cas cyclisation,
(2) - réduction du groupe nitro en amine,
(3) - acylation de la fonction amine obtenue selon (2),
(4) - alcoylation du carbamate obtenu selon (3), éventuellement suivie d'une hydrogénolyse,
(5) - saponification de la fonction ester du cycle pyrrole,
(6) - condensation du NN'-cyclohexylurée sur la fonction acide obtenue selon (5),
(7) - réduction de la fonction acide obtenue selon (5) en aldéhyde,
(8) - condensation d'Horner-Emmons sur la fonction aldéhyde obtenue selon (6),
(9) - substitution électrophile sur le cycle pyrrole,
(10) - réaction de cyclisation intramoléculaire entre les deux cycles aromatiques.

La création d'un troisième cycle, selon cette dernière transformation, joignant les noyaux aromatiques, est réalisée à partir de chaînes fonctionnalisées ou de groupements réactifs fixés, éventuellement à partir d'une ou plusieurs des transformations citées ci-dessus, sur chacun des noyaux phényle et pyrrole, qui permettent une fermeture de type intramoléculaire.

Compte-tenu de leurs intéressantes propriétés antimitotiques, les composés phénylpyrroles, représentés par la formule générale I, s'avèrent utiles en tant que médicaments. L'invention s'étend également aux compositions pharmaceutiques caractérisées en ce qu'elles contiennent en tant que principe actif un composé de formule générale I.

D'autres caractéristiques et avantages de la présente invention apparaîtront à la lecture de la description faite ci-après, notamment à l'aide d'un certain nombre d'exemples donnés à simple titre d'illustration.

Les composés phénylpyrroles suivants ont été préparés selon le mode opératoire décrit ci-dessous en II, à partir des nitrostyrènes correspondants, euxmêmes synthétisés préliminairement selon la méthode I.

I <u>Préparation des nitrostyrènes</u> :

L'aldéhyde (2 mmoles) est dissous dans le mélange tampon : acide acétique glacial 10 ml / acétate d'ammonium 600 mg. On ajoute 1,4 équivalent de nitroéthane et on porte au reflux. Au bout de 3 à 4 heures, l'aldéhyde a disparu et il se forme un produit principal moins polaire. On verse le mélange réactionnel dans 20 ml d'eau glacée et on extrait par du chlorure de méthylène. La phase organique est lavée 2 foie à l'eau, 2 fois à l'eau carbonatée puis séchée sur sulfate de sodium et enfin évaporée. On isole le nitrostyrène sur colonne de silice (éluant : $CH_2Cl_2$/hexane ; 4/1).

II <u>Méthode générale de préparation des composes de type phénylpyrrole à partir des nitrostyrènes correspondants</u> :

Le nitrostyrène (20 mmoles) est dissous dans 50 ml d'un mélange de tétrahydrofuranne distillé / tertiobutanol absolu (2/1). On ajoute 1,5 équivalent de DBU puis goutte à goutte et à température ambiante 1,2 équivalent (2,7 ml) d'isocyanoacétate d'éthyle. Le mélange réactionnel est porté à 40-50 °C et agité

pendant 3 à 4 heures (sauf précision). La réaction est totale et un produit majoritaire moins polaire que le nitrostyrène de départ se forme. Les phénylpyrroles ainsi obtenus se révèlent en rose sur plaque chromatographique par pulvérisation du mélange vanilline / HCl concentré. Les solvants sont évaporés et on sépare directement le produit sur colonne de silice (éluant : $CH_2Cl_2$/hexane ; 4/1).

Remarque : Le remplacement de l'isocyanoacétate d'éthyle par l'isocyanoacétate de méthyle donne l'ester méthylique avec le même rendement.

Exemple 1

Méthyl-4-phényl-3 pyrrolecarboxylate-2 d'éthyle.

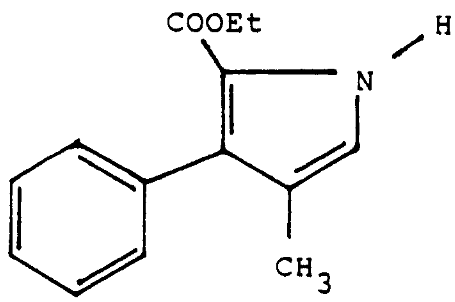

(1)

La synthèse est réalisée à partir du phényl-1-nitro-2-propènel-1selon la méthode générale II. Le méthyl-4-phényl-3 pyrrolecarboxylate-2 d'éthyle, ainsi obtenu, est purifié sur colonne de silice pour donner une poudre blanche (3,9 g ; Rdt = 85%).

. PF : 62-64 °C
. IR ($CHCl_3$) : 3480, 3300, 2990, 2940, 1675, 1490, 1460, 1290 $cm^{-1}$
. UV(méthanol) λmax: 276,4 220,3 207,2 nm
  ε : 13900 11700 12800 $mole^{-1}xlxcm^{-1}$
. SM : m/z : 229 ($M^{+\cdot}$), 200, 183, 156, 155, 154
. Microanalyse $C_{14}H_{15}NO_2$

| | | | | | | |
|---|---|---|---|---|---|---|
| calculée % :<br>trouvée % : | C | 73,34<br>72,85 | H | 6,59<br>6,38 | N | 6,10<br>6,06 |

. RMN $^1H$ ($CDCl_3$, 400 MHz)
1.1 (3H, t J=7Hz, $CH_2CH_3$)
2 (3H, s, $CH_3$)
4.05 (2H, q J=7Hz, $CH_2CH_3$)
6.7 (1H, d, H pyrrolique)
7.15-7.4 (5H, m, Ph)
9.15 (1H, br, NH)

Exemple 2

Méthyl-4 (nitro-3' phényl)-3 pyrrolecarboxylate-2 de méthyle

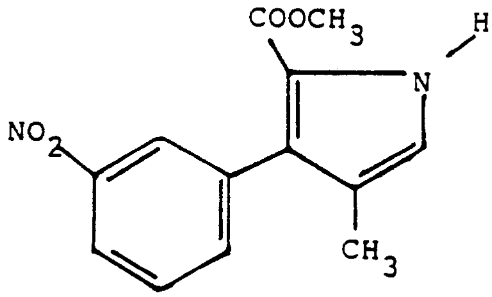

(2)

Il est obtenu à partir du (nitro-3' phényl)-1 nitro-2 propène-1 selon le mode opératoire II. On obtient 4,2 g de composé (2) (Rdt : 80%) qui peut être recristallisé dans un mélange acétate d'éthyle/hexane.

. PF : 146 °C
. IR ($CHCl_3$) : 3460, 1690 ($COOM_e$) 1540, 1360, 1160 $cm^{-1}$
. UV (éthanol absolu)
  λmax : 270,2 205,5 nm ε :16700 13700 mole $xl^{-1}xcm^{-1}$
. SM : m/z : 260 ($M^{+\cdot}$), 228, 153
. Microanalyse : $C_{13}H_{12}N_2O_4$

| | | | | | | | | |
|---|---|---|---|---|---|---|---|---|
| calculée % :<br>trouvée % | C | 59,99<br>60,07 | H | 4,65<br>4,84 | N | 10,77<br>10,82 | O | 24,59<br>24,82 |

. RMN¹H ($CDCl_3$, 200 MHz)
2.03 (3H, s, $OCH_3$)
3.75 (3H, s, $CH_3$)
6,91 (1H, d J=2Hz, H pyrrolique)
7,63 (1H, t J=8Hz, $H_5$')
7,8 (1H, d J=8Hz, $H_6$')
8,28 (1H, d J=8Hz, $H_4$')
8,33 (1H, s, $H_2$')
9,33 (1H, br, NH)

Exemple 3

Méthyl-3 pyrrolo[2,3c]quinolin-one-4(5H)

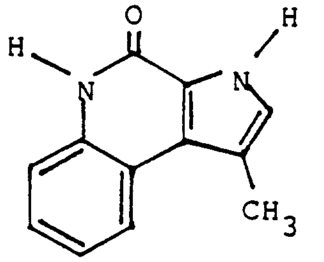

(4)

La synthèse est réalisée en plusieurs étapes, à partir du (nitro-2'phényl)-1 nitro-2 propène-1 préalablement préparé selon la méthode I :

a) Synthèse du méthyl-4 (nitro-2' phényl)-3 pyrrolecarboxylate-2 d'éthyle (3)

Elle est réalisée à partir du (nitro-2' phényl)-1 nitro-2 propène-selon le mode opératoire décrit en II.

La purification sur colonne de silice donne 5,4 g d'une poudre jaune (Rdt = 98%). La recristallisation du produit dans un mélange DMSO/$H_2O$ permet d'obtenir des cristaux jaunes, (PF : 116°C)

b) Synthèse du méthyl-3 pyrrolo[2,3c]quinolin-one-4(5H) : (4)

Dans 10 ml de méthanol sont mélangés : 274 mg (1 mmole) de (3), 1 g de $FeCl_3 . 6H_2O$ (et 50 mg de charbon activé). On porte le milieu réactionnel au reflux pendant 10 minutes, puis on ajoute goutte à goutte, 1,5 équivalent d'hydrazine. Le reflux est maintenu pendant 12 heures. Le produit attendu (4) apparaît, ainsi que du (amino-2' phényl)-3 méthyl-4 pyrrolecarboxylate d'éthyle, composé (5).

Le catalyseur est filtré sur célite et on laisse refroidir la solution recueillie dans laquelle le produit de cyclisation cristallise. On recueille 80 mg de cristaux blanc-cassé (Rdt = 40%). La solution contenant le produit de réduction est évaporée et on purifie celui-ci par chromatographie sur colonne de silice (110 mg de (5) sont obtenus, Rdt = 45%).

. Analyses de (4)
. PF : 254 - 256°C
. IR (nujol) : 1620 $cm^{-1}$
. UV(DMSO) : λmax : 320 306 258 nm
  ε : 11100 9700 8200 $mole^{-1}xlxcm^{-1}$
. SM: m/z : 198 ($M^{+\cdot}$), 197, 179, 169
. RMN$^1$H ($CD_3OD$, 60 MHz)
  2.5 (3H, s, $CH_3$)
  7.1-7.5 (5H, m, Ph, H pyrrolique)
  8.1 (1H, m, CONH)
. Analyses de (5)
. SM: m/z : 224 ($M^{+\cdot}$), 199 (M-OEt)
. RMN$^1$H ($CDCl_3$, 60 MHz)
  1.06 (3H, t J = 7Hz, $CH_2CH_3$)
  1.91 (3H, s, $CH_3$)
  3.38 (2H, br, $NH_2$)
  4.13 (2H, q J = 7Hz, $CH_2CH_3$)
  6.53-7.33 (5H, m, Ph, H pyrrolique)

Exemple 4

(N-benzyloxycarbonylglycylamido-2' phényl)-3 méthyl-4 pyrrolecarboxylate-2 d'éthyle

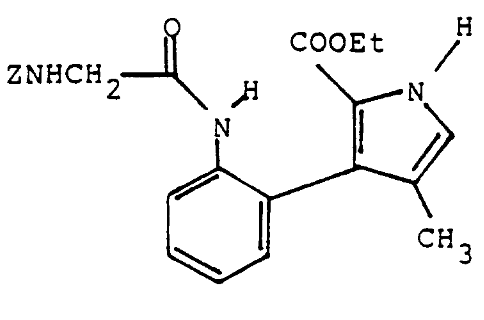

(6)

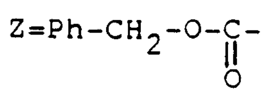

La synthèse du composé (6) est réalisée à partir de l'(amino-2' phényl)-3 méthyl-4 pyrrolecarboxylate-2 d'éthyle (5) obtenu dans l'exemple 3.

843 mg (4,05 mmoles) de glycine N-benzylcarbamate (Z-glycine) sont dissous dans 80 ml de tétrahydrofuranne anhydre. A la solution placée sous argon et refroidie à -15°C, on ajoute 580 mg (4,05 mmoles) de N-méthylmorpholine puis 570 mg de chloroformate d'isobutyle goutte à goutte. Après 10 minutes d'agitation à -15°C, on verse goutte à goutte une solution de 500 mg (2,23 mmoles) de (5) dans

50 ml de tétrahydrofuranne. La température du milieu réactionnel est maintenue 5 minutes à -15 °C ; puis on revient à température ambiante. La réaction est totale en 1 heure.

Le sel de N-méthylmorpholine est filtré et lavé par du tétrahydrofuranne. Après évaporation du tétrahydrofurannè, on reprend par de l'acétate d'éthyle ; la partie insoluble est filtrée ; on lave la phase organique successivement par de l'eau acidulée (HCl 0,1N), de l'eau saturée en carbonate de sodium puis en chlorure de sodium. Après séchage sur sulfate de sodium et évaporation, on isole 700 mg de (diméthoxy-4',5' N-benzyloxycarbonylglycylamido-2' phényl)-3 méthyl-4 pyrrolecarboxylate d'éthyle (Rdt = 79%). La recristallisation dans un mélange acétate d'éthyle/hexane donne des cristaux jaunes pâles.

. PF : 124 °C
. IR ($CHCl_3$) : 3460, 1690, 1590, 1510, 1290 $cm^{-1}$
. UV (DMSO) : λmax : 258 nm
  ε : 11300 $mole^{-1}xlxcm^{-1}$ (éthanol absolu)λmax : 276 (épaulement), 239, 207 nm
. SM : m/z : 435 ($M^{+\cdot}$) , 244, 243, 199
. Microanalyse : $C_{24}H_{25}N_3O_5$

| | | | | | | | | |
|---|---|---|---|---|---|---|---|---|
| calculée % :<br>trouvée % : | C | 66,19<br>65,97 | H | 5,78<br>5,63 | N | 9,65<br>9,68 | O | 18,37<br>18,65 |

. RMN¹H ($CDCl_3$, 80 MHz)
0.95 (3H, t J = 7Hz, $CH_2CH_3$)
1.8 (3H, s, $CH_3$)
3.72 (2H, d, J = 5Hz, $CH_2NH$)
4 (2H, q J = 7Hz $CH_2CH_3$)
5 (2H, s, $CH_2$ Ph)
5,66 (1H, br, $CH_2NH$)
6.61 (1H, d J = 2Hz, H pyrrolique)
7-7.25 (8H, m, Ph)
7.87 (1H, br, s, Ph NH)
8.2 (1H, d J = 7Hz, $H_{3'}$)

Exemple 5

Diméthyl-1,4 (nitro-2' phényl)-3 pyrrole N,N'-cyclohexyluréido-carboxamide-2

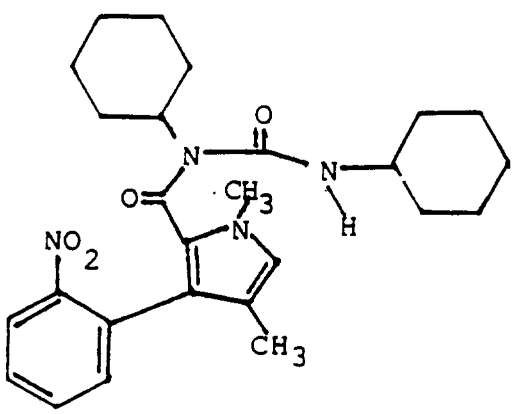

(10)

Il est synthétisé à partir du méthyl-4 (nitro-2' phényl)-3 pyrrolecarboxylate-2 d'éthyle (3) préparé selon le mode opératoire décrit précédemment dans l'exemple 3.

a) Synthèse du diméthyl-1,4(nitro-2' phényl)-3 pyrrolecarboxylate-2 d'éthyle (7).

1 g (3,65 mmoles) de (3) est dissous dans 20 ml de tétrahydrofuranne anhydre. Le mélange est refroidi dans la glace et on forme l'anion en ajoutant un équivalent d'hydrure de sodium. Après 15 minutes d'agitation à froid, on passe à température ambiante. On ajoute alors 1,3 équivalent de paratoluènesulfonate de méthyle et on chauffe le milieu réactionnel à 50 °C pendant 3 heures. La réaction est quantitative. Il se

forme un produit moins polaire que (3). On laisse refroidir, filtre sur célite et lave celle-ci par du chlorure de méthylène. Le produit est obtenu sous forme d'une huile jaune après purification sur colonne de silice (éluant : $CH_2Cl_2$/hexane ; 3/2). On obtient 0,95 g de (7) (Rdt : 90%).

b) Synthèse de l'acide diméthyl-1,4 (nitro-2' phényl)-3 pyrrolecarboxylique-2 (8).

(7) est saponifié dans un mélange méthanol/soude 30% (5/1), à chaud (60 °C) pendant 2 heures. On acidifie la solution jusqu'à pH = 3 en refroidissant dans la glace. L'acide précipite et est extrait par du chlorure de méthylène. La phase organique est lavée par de l'eau saturée en chlorure de sodium et séchée sur sulfate de sodium. On purifie le produit sur colonne de silice (éluant : $CH_2Cl_2$/MeOH 95/5). Le composé (8) attendu est isolé sous forme de cristaux jaunes recristallisés dans un mélange éther éthylique/hexane, PF 205 °C, (190 mg ; Rdt : 73%).

c) Synthèse du diméthyl-1,4 (nitro-2' phényl)-3 pyrrole N,N'-cyclohexyluréidocarboxamide-2 (10).

78 mg (1,5 équivalent) de chlorhydrate de l'ester éthylique de la glycine et 0,08 ml (1,5 équivalent) de triéthylamine anhydre sont dissous dans 5 ml d'acétonitrile. On ajoute 100 mg (0,38 mmole) de (8) et refroidit la solution à -5 °C.

117 mg de dicyclohexylcarbodiimide (DCCI) sont alors introduits ; le mélange réactionnel est agité 4 heures à -5 °C puis 12 heures à température ambiante. Sur plaque chromatographique, on voit apparaître 2 produits moins polaires que l'acide (8). Il s'agit du composé attendu (10) et du diméthyl-1,4 (nitro-2' phényl)-3 pyrrole éthylate de glycylcarboxamide-2, (11).

L'acide n'est pas totalement consommé. On évapore le solvant de réaction et reprend par l'acétate d'éthyle. La partie insoluble est filtrée ; la phase organique est lavée successivement par de l'eau saturée en carbonate de sodium, une solution 1 N d'acide chlorhydrique, de l'eau saturée en chlorure de sodium. Après séchage sur sulfate de sodium et évaporation, on obtient 160 mg d'un mélange brut de 2 produits qui sont séparés sur colonne de silice ; ce qui donne : (10) : 70 mg (Rdt = 39%)

. SM : m/z : 466 ($M^{+\cdot}$), 341, 243, 199
. RMN$^1$H ($CDCl_3$, 80MHz)
  1-1.87 (22H, br, H cyclohexyle)
  1.8 (3H, s, $CH_3$)
  3.6 (3H, s, N $CH_3$)
  6.47 (1H, s, H pyrrolique)
  7.2-7.7, (3H, m, Ph)
  7.87 (1H, d J = 7Hz $H_3$,)

Exemple 6

Butyl-5 diméthyl-1,4 (dioxa-1,4 éthylcarbaldéhyde-2) -2 (N-benzyloxycarbonyl N-méthylamino-2' phényl)-3 pyrrole

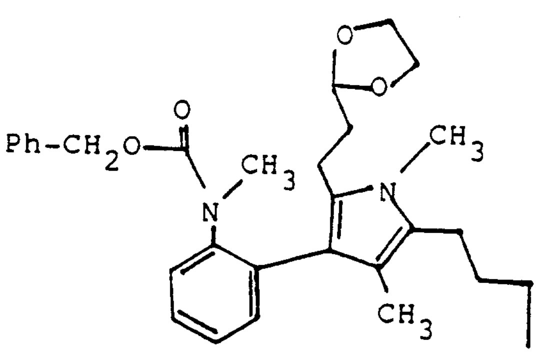

(22)

Il est obtenu à partir du diméthyl-1,4 (nitro-2' phényl)-3 pyrrolecarboxylate-2 d'éthyle (7) dont la préparation a été décrite dans l'exemple 5. La synthèse du composé (22) nécessite les étapes suivantes :

a) Synthèse du diméthyl-1,4 hydroxyméthyl-2 (nitro-2' phénylpyrrole (12).

On dissout 200 mg de (7) dans 30 ml de chlorure de méthylène anhydre et on refroidit à -78°C dans un bain (acétone/carboglace). 2,8 ml (4 équivalents) de dibal 1M dans le toluène sont ajoutés goutte à goutte. L'agitation est maintenue 2 heures à -78°C puis on sort le ballon du milieu réfrigérant et on verse 50 ml d'une solution aqueuse de carbonate de sodium (10%). Une émulsion importante apparaît que l'on élimine par filtration sur célite. La phase organique limpide recueillie est lavée par de l'eau saturée en chlorure de sodium et séchée sur sulfate de sodium. L'alcool (12) est purifié sur colonne de silice, (120 mg ; Rdt 71%).

b) Synthèse du diméthyl-1,4 (nitro-2' phényl)-3 pyrrolecarbaldéhyde (13).

100 mg de $MnO_2$ préparé selon la méthode d'Attenburrow sont mis en suspension dans un mélange tétrachlorure de carbone/chlorure de méthylène (4/1) contenant 500 mg d'alcool (12). On agite le milieu réactionnel à température ambiante pendant 1 heure ½. L'agent d'oxydation est alors filtré sur célite. On obtient 230 mg de composé (13), (Rdt : 46%). Ce composé est purifié par Chromatographie sur colonne de silice (éluant : acétate d'éthyle/hexane : 1/1). Il a l'aspect d'une huile jaune.

c) Synthèse du diméthyl-1,4 (nitro-2' phényl)-3 (éthylate de carboxy-2 vinyl)-2 pyrrole (14).

470 mg (3 équivalents) d'hydrure de sodium (en poudre à 97%) sont mis en suspension sous argon dans 40 ml d'éther diméthylique de l'èthylène glycol (DME) anhydre. On refroidit dans la qlace et on ajoute 3,9 ml (3 équivalents) de triéthylphosphonoacétate dilué dans 5 ml de DME. Un dégagement gazeux se produit et le mélange réactionnel est agité à température ambiante jusqu'à consommation totale de l'hydrure. Dans la solution réactionnelle devenue limpide, on verse 1,6 g de (13) dissous dans 30 ml de DME.

Après 6 heures d'agitation à température ambiante, le mélange réactionnel est filtré sur célite. On évapore le DME et reprend par du chlorure de méthylène. La phase organique est lavée par une solution aqueuse de chlorure de sodium jusqu'à disparition de l'émulsion produite, puis séchée sur sulfate de sodium. On obtient après évaporation du solvant, 4 g de produit brut qui, une fois purifié sur colonne de silice (éluant : acétate d'éthyle/hexane ; 2/3), fournit une poudre jaune de composé (14), (2 g : Rdt 97%).

d) Synthèse du (butène-1-yl)-5 diméthyl-1,4 (éthylate de carboxy-2 vinyl)-2 (nitro-2' phényl)-3 pyrrole : (15)

4 g (12,7 mmoles) de (14) sont mis en solution à 0°C dans 60 ml d'un mélange tétrahydrofuranne/HCl à 6% (50 ml de tétrahydrofuranne/10 ml d'acide chlorhydrique à 36%). 1,5 ml (3 équivalents) de butyraldéhyde sont ajoutés goutte à goutte. Le mélange réactionnel est agité pendant 7 heures à froid. On neutralise la solution acide par une solution aqueuse saturée en carbonate de sodium puis on extrait le produit par de l'acétate d'éthyle. La phase organique est lavée par de l'eau carbonatée (10%) puis séchée sur sulfate de sodium. Le composé (15) obtenu est purifié sur colonne de silice (éluant : acétate d'éthyle/hexane ; 1/4). On obtient des cristaux rouges (1,8 g ; Rdt : 38%).

e) Synthèse du (butène-1-yl)-5 diméthyl-1,4 (hydroxyméthyl-2 vinyl)-2 (nitro-2' phényl)-3 pyrrole : (16).

4 g du composé (15) sont réduits par 3 équivalents d'hydrure de diisobutylaluminium. On isole le composé (16) sous forme d'une poudre rouge (3 g ; Rdt = 85%).

f) Synthèse du (butène-1-yl)-5 diméthyl-1,4 (nitro-2' phényl)-3 (vinylcarbaldéhyde-2)-2 pyrrole : (17)

3 g de (16) sont oxydés en 1 heure ½ par 20 g de dioxyde de manganèse, préparé selon la méthode d'Attenburrow dans 60 ml de tétrachlorure de carbone. Après filtration du mélange réactionnel sur célite et purification du produit sur colonne de silice, on isole des cristaux rouge-orangé de composé (17), (2 g ; Rdt = 67%).

g) Synthèse du (butène-1-yl)-5 diméthyl-1,4 (nitro-2' phényl)-3 (dioxa-1,4 vinylcarbaldéhyde-2)-2 pyrrole : (18).

2,6 g (8 mmoles) de (17), 4,5 ml (10 équivalents) d'éthylèneglycol, ainsi qu'une quantité catalytique (20 mg) de tosylate de pyridinium sont mis en solution dans 60 ml de benzène. Le mélange réactionnel est porté au reflux pendant 4 heures et vire au vert. (L'eau est éliminée azéotropiquement à l'aide d'un Deanstark). Il se forme un produit moins polaire que l'aldéhyde. Après refroidissement, on lave 3 fois la phase benzénique par une solution aqueuse de carbonate de sodium et on la sèche sur sulfate de sodium. Le produit brut obtenu après évaporation est purifié sur colonne d'alumine (éluant : acétate d'éthyle/ hexane ; 2/3) ; il a l'aspect d'une pâte rouge (nous avons noté une légère instabilité du produit lors de son passage sur silice). On obtient 2,2 g de composé (18), (Rdt = 75%).

h) Synthèse de l'(amino-2'phényl)-3 (butène-1-yl)-5 diméthyl-1,4 (dioxa-1,4 vinylcarbaldéhyde-2)-2 pyrrole : (19).

Le composé (18) est réduit par l'hydrazine dans l'éthanol en présence de nickel de Raney. 2 g de composé (18) donnent 1,7 g d'une pâte jaune de composé (19), (Rdt = 93%).

i) Synthèse de l'(amino-2'phényl)-3 butyl-5 diméthyl-1,4 (dioxa-1,4 éthylcarbaldéhyde-2)-2 pyrrole : (20).

1,9 g de (19) est réduit dans 40 ml d'éthanol en présence de 400 mg de palladium sur charbon (10%), sous une atmosphère d'hydrogène. Après 12 heures de réaction, le catalyseur est filtré sur célite et le produit est purifié sur colonne d'alumine (éluant : acétate d'éthyle/hexane ; 2/3). On isole le composé (20) sous forme d'une pâte jaune (1,6 g ; Rdt = 83%).

j) Synthèse du (benzyloxycarbonylamino-2'phényl)-3 butyl-5 diméthyl-1,4 (dioxa-1,4 éthylcarbaldéhyde-2)-2 pyrrole : (21).

1,6 g de (20) est dissous dans 150 ml d'éther diméthylique de l'éthylène glycol (DME) anhydre. La solution est refroidie dans la glace et on ajoute 5,5 ml (20 équivalents) de pyridine distillée. On introduit ensuite goutte à goutte en deux heures, une solution de 1,2 ml (1,8 équivalent) de chloroformate de benzyle dans 20 ml de DME (l'introduction lente du réactif donne un meilleur rendement). Le milieu réactionnel est agité une heure à température ambiante puis on filtre le chlorhydrate de pyridinium formé. On évapore le DME et reprend par l'acétate d'éthyle ; la phase organique est lavée par des solutions aqueuses de chlorure de sodium et de carbonate de sodium saturées puis, séchée sur sulfate de sodium et évaporée. On obtient le composé (21) sous forme d'une huile jaune que l'on purifie sur colonne d'alumine - (éluant : acétate d'éthyle/hexane ; 2/3), (Rdt = 67%).

k) Synthèse du butyl-5 diméthyl-1,4 (dioxa-1,4 éthylcarbaldéhyde-2)-2 (N-benzyloxycarbonyl N-méthylamino-2' phényl)-3 pyrrole : (22).

1,5 g de (21) est mis en solution sous argon dans 100 ml de DME anhydre. Après avoir refroidi à -20 °C (glace/chlorure de sodium), on forme l'anion de (21) en ajoutant 453 mg (1,3 équivalent) de tertiobutylate de potassium. La solution jaune vire au rouge-brun et un précipité apparaît. Le mélange réactionnel est agité 30 minutes à -20 °C puis 0,6 ml (3 équivalents) d'iodure de méthyle est introduit goutte à goutte. L'agitation est poursuivie une heure à -20 °C. On élimine le précipité par filtration sur célite ; le DME est évaporé et on reprend par de l'acétate d'éthyle. La phase organique une fois lavée par une solution saline de chlorure de sodium est séchée sur sulfate de sodium et évaporée. Le produit est purifié sur colonne d'alumine (éluant : acétate d'éthyle/hexane ; 2/3). On isole une huile jaune. Le composé (22), ainsi obtenu, est un mélange de diastéréoisomères (1,1 g ; Rdt = 71%).

. IR ($C_6H_6$) : 2980, 2940, 2880, 1710, 1450, 1390, 1350, 1160 $cm^{-1}$
. UV (DMSO) : λmax : 257 nm (éthanol absolu):
  max : 208 nm
. SM : m/z : 490 ($M^{+\cdot}$) , 447 ($M-CH_2CH_2CH_3$), 403, 390, 220, 205
. RMN$^1$H ($C_6D_6$, 200 MHz)
  0,87 (3H, t J = 7Hz, $CH_2CH_3$)
  1.29 (4H, m, $CH_2CH_2CH_3$)
  1.91 (5H, br, s, $CH_2CHOO$, $CH_3$)

2.41 (2H, br, t, $\underline{CH_2}CH_2CH_2$)
2.78 (2H, m, $\underline{CH_2}CH_2CHOO$)
3.03 (3H, s, $NCH_3$)
3.09 (3H, s, $NCH_3$)
3.41 (4H, m, $OCH_2CH_2O$)
4.65 (1H, t, CHOO)
5.3 (2H, br s, Ph $\underline{CH_2}$)
6.85-7.9 (4H, m, $\underline{Ph}$)

Exemple 7

Butyl-5 diméthyl-1,4 (dioxa-1,4 éthylcarbaldéhyde-2)-2 (N-methylamino-2'phényl)-3 pyrrole.

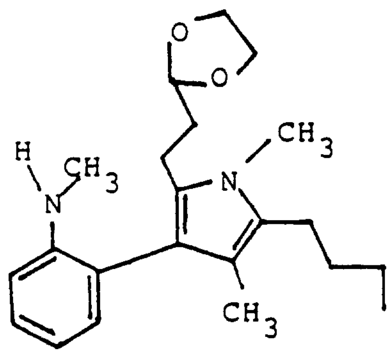

(23)

Ce composé est obtenu par hydrogénolyse du groupement carbamate de benzyle du composé (22). L'hydrogénolyse est réalisée sous une atmosphère d'hydrogène, en présence de palladium sur charbon (à 10%), dans l'acétate d'éthyle. Au bout de 15 heures d'agitation, on filtre le catalyseur sur célite, puis le produit est purifié sur colonne d'alumine (éluant : acétate d'éthyle/hexane ; 3/7). 1 g de composé (22) fournit après déprotection 500 mg d'un produit huileux rouge-brun (23) (Rdt = 70%).

. IR ($C_6H_6$) : 3350 (massif), 2900, 2850, 1680, 1600, 1360, 1120 $cm^{-1}$
. UV (DMSO) : max : 306 (épaulement) 257 nm
. SM : m/z : 356 ($M^{+\cdot}$), 269
. RMN$^1$H ($C_6D_6$, 200 MHz)
0,87 (3H, t J = 7Hz, $CH_2\underline{CH_3}$)
1.33 (4H, m, $\underline{CH_2CH_2}CH_3$)
1.95 (2H, m, $\underline{CH_2}CHOO$)
2.03 (3H, s, $CH_3$)
2.45 (3H, d J = 5Hz, $\underline{CH_3}NH$)
2.47 (2H, t J = 7Hz, $\underline{CH_2}CH_2CH_2CH_3$)
2.78 (2H, m, $\underline{CH_2}CH_2CHOO$)
3.16 (3H, s, $NCH_3$)
3.39 (4H, m, $OCH_2CH_2O$)
4.03 (1H, q J = 5Hz, $CH_3\underline{NH}$)
4.64 (1H, t J = 5Hz, $CH_2\underline{CH}OO$)
6.74 (1H, d J = 8Hz, $H_6$')
6.95 (1H, t J = 8Hz, $H_4$')
7.36 (2H, m, $H_3$', $H_5$')

Etude biologique

L'évaluation de l'activité biologique des produits synthétisés a été effectuée en mettant en oeuvre plusieurs tests biologiques : Après avoir étudié l'interaction de ces composés avec la tubuline (inhibition de la polymérisation ou de la dépolymérisation) et de cette façon, avoir mis en évidence leur activité en tant que poison du fuseau mitotique, il a été procédé à des études de cytotoxicité.

Les études de cytotoxicité ont été réalisées, d'une part sur la lignée véro (cellule normale de rein de singe) Tableau I, et d'autre part, sur la lignée KB (carcinome humain du nasopharynx) Tableau II, selon la méthode suivante :

Matériel et méthode.

Les produits ont été déposés au moment de la mise en culture, laquelle est maintenue pendant 72 heures. Les produits sont testés à 100, 80, 40, 20 et 10 µm. A ces concentrations, le D.M.S.O. utilisé pour dissoudre les composés n'excède pas 1% dans les cultures et est non toxique. Les pourcentages d'inhibition de la prolifération cellulaire ont été évalués par une méthode colorimétrique au cristal violet, relativement à des cultures témoins contenant du D.M.S.O. ( ≦ 1%)

Tableau I

| Cellules Véro | produits soumis aux essais de cytostase % d'inhibition de la prolifération. | | | | | | |
|---|---|---|---|---|---|---|---|
| Concentration en µm | Composés N° | | | | | | |
| | (1) | (2) | (4) | (6) | (10) | (22) | (23) |
| 100 | 63 | 77 | 69-84 | 78 | 100 | 54 | 80 |
| 80 | 46 | 75 | 49-70 | 48 | 100 | 54 | 69 |
| 40 | 20 | 38 | 45 | 4 | 25 | 0 | 25 |
| 20 | 0 | 3 | 0 | 0 | 0 | 0 | 0 |
| 10 | 0 | 0 | 0 | 0 | 0 | 0 | 0 |

Tableau II

| Cellules KB | produits soumis aux essais de cytostase % d'inhibition de la prolifération | | | | | | |
|---|---|---|---|---|---|---|---|
| Concentration en µm | Composés N° | | | | | | |
| | (1) | (2) | (4) | (6) | (10) | (22) | (23) |
| 100 | 43 | 72 | 68 | 68 | 45 | 59 | 100 |
| 80 | 35 | 50 | 60 | 52 | 27 | 44 | 94 |
| 40 | 0 | 10 | 100 | 31 | 0 | 0 | 81 |
| 20 | 0 | 0 | 72 | 22 | 0 | 0 | 66 |
| 10 | 0 | 0 | 0 | 0 | 0 | 0 | 48 |
| 5 | x | x | x | x | x | x | 4 |

Résultats :

Les composés de la présente invention, en particulier ceux des exemples 4, 6 et surtout 23, se révèlent particulièrement intéressants. Ils montrent une certaine sélectivité à faible concentration à l'égard de la lignée KB.

La présente invention concerne également des compositions pharmaceutiques contenant à titre de principe actif au moins un composé représenté par la formule générale I associé à tout véhicule pharmacologique classique, permettant son administration en particulier par voie orale et par voie parentérale.

## Revendications

**1.** Composés caractérisés en ce qu'ils sont représentés par la formule générale (I) :

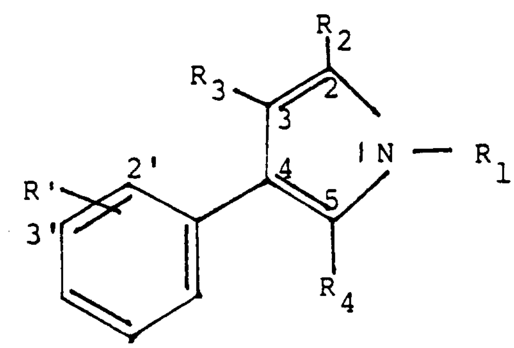

(I)

dans laquelle

- $R_1$, $R_2$ et $R_3$ indépendamment l'un de l'autre, sont représentés par :
  (1) - H
  (2) - alcoyle $C_{1-6}$,
- R' situé en position 2' ou 3' est représenté par :
  (1) - H
  (2) - $NO_2$
  (3) -

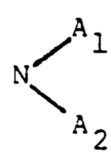

dans lequel $A_1$ et $A_2$ indépendamment l'un de l'autre, représentent :
(a) - H
(b) - alcoyle $C_{1-4}$,
(c) - alcoylène en $C_{1-6}$ ou alcénylène en $C_{1-6}$ qui se cyclise sur l'atome de C en position 5 du cycle pyrrole,
(d) -

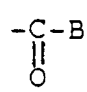

dans lequel B représente :
- phénylalcoxy en $C_{1-4}$,
- N-benzyloxycarbonylaminoalcoyle $C_{1-4}$,

- alcoylène en $C_{1-6}$ ou alcénylène en $C_{1-6}$ qui se cyclise sur l'atome de C en position 5 du cycle pyrrole,

- $R_4$ est représenté par :

(1) - hétérocycloalcoyl- (alcoyle $C_{1-4}$) contenant 5 ou 6 chaînons,

(2) -

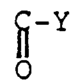

dans lequel Y représente :

(a) - alcoxy $C_{1-4}$,

(b) - NN'-cycloalcoyl-(uréido) contenant 5 ou 6 chaînons,

(c) - amino cyclisable en position R'.

**2.** Les composés caractérisés selon la revendication 1, en ce qu'il s'agit de :

- méthyl-4 phényl-3 pyrrolecarboxylate-2 d'éthyle,
- méthyl-4 (nitro-3' phényl)-3 pyrrolecarboxylate-2 de méthyle,
- méthyl-3 pyrrolo [2,3c] quinolin-one-4(5H),
- (N-benzyloxycarbonylglycylamido-2'phényl)-3 méthyl-4 pyrrolecarboxylate-2 d'éthyle,
- diméthyl-1,4 (nitro-2' phényl)-3 pyrrole N,N'-cyclohexyluréidocarboxamide-2,
- butyl-5 diméthyl-1,4 (dioxa-1,4 éthylcarbaldéhyde-2)-2 (N-benzyloxycarbonyl N-méthylamino-2' phényl)-3 pyrrole,
- butyl-5 diméthyl-1,4 (dioxa-1,4 éthylcarbaldéhyde-2)-2 (N-méthylamino-2' phényl)-3 pyrrole.

**3.** Procédé de préparation des composés représentés par la formule générale (I) selon les revendications 1 et 2, caractérisé en ce que l'on fait réagir un nitro-styrène de formule II

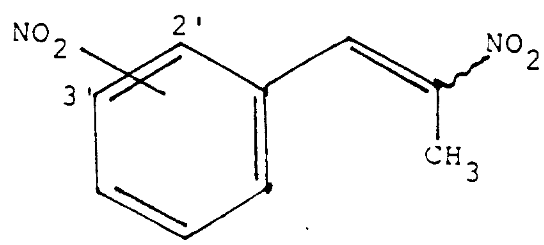

(II)

dans laquelle :

le groupe $NO_2$ est situé en position 2' ou 3', avec un isocyanoacétate d'alcoyle de formule III

$R-O-CO-CH_2-NC$ (III)

dans laquelle :

R est un groupement éthyle ou méthyle,

et dans le cas où le composé I' est obtenu, en ce qu'on le transforme en d'autres composés représentés par la formule générale I

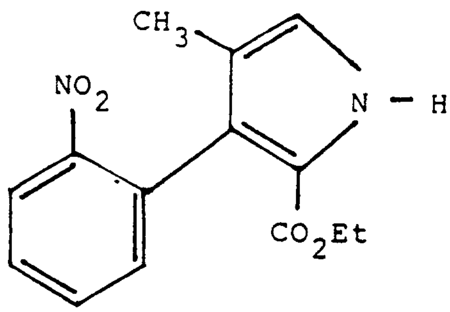

I'

par la mise en oeuvre d'au moins une des opérations suivantes :

(1) - réduction simultanée du groupe nitro du cycle aromatique et de la fonction ester du cycle pyrrole et dans ce cas cyclisation,
(2) - réduction du groupe nitro en amine,
(3) - acylation de la fonction amine obtenue selon (2),
(4) - alcoylation du carbamate obtenu selon (3), éventuellement suivie d'une hydrogénolyse,
(5) - saponification de la fonction ester du cycle pyrrole,
(6) - condensation du NN'-cyclohexylurée sur la fonction acide obtenue selon (5),
(7) - réduction de la fonction acide obtenue selon (5) en aldéhyde,
(8) - condensation d'Horner-Emmons sur la fonction aldéhyde obtenue selon (6),
(9) - substitution électrophile sur le cycle pyrrole, et
(10) - réaction de cyclisation de type intramoléculaire entre les deux noyaux aromatiques.

**4.** Les composés représentés par la formule générale I selon les revendications 1 et 2, à titre de médicaments.

**5.** Compositions pharmaceutiques caractérisées en ce qu'elles contiennent en tant que principe actif au moins un composé de formule générale I selon l'une des revendications 1 et 2.

## Claims

**1.** Compounds, characterized in that they are represented by the general formula (I):

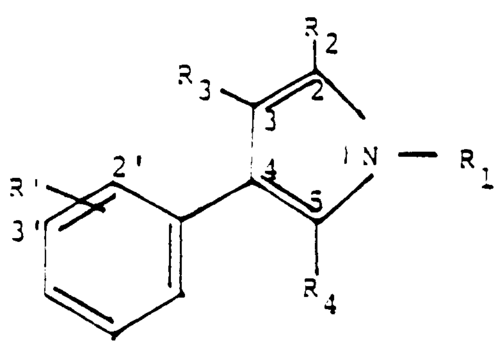

(I)

in which

- $R_1$, $R_2$ and $R_3$, independently of one another, are represented by:
  (1) - H
  (2) - $C_{1-6}$ alkyl,
- R' situated at the 2'-or 3'-position is represented by:
  (1) - H
  (2) - $NO_2$

(3) -

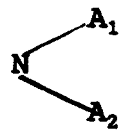

in which $A_1$ and $A_2$, independently of one another, represent:

(a) - H

(b) - $C_{1-4}$ alkyl,

(c) - $C_{1-6}$ alkylene or $C_{1-6}$ alkenylene which cyclizes onto the C atom at the 5-position of the pyrrole ring,

(d) -

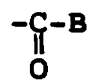

in which B represents:

- phenyl($C_{1-4}$ alkoxy),
- N-benzyloxycarbonylamino-($C_{1-4}$ alkyl),
- $C_{1-6}$ alkylene or $C_{1-6}$ alkenylene which cyclizes onto the C atom at the 5-position of the pyrrole ring,

- $R_4$ is represented by:

(1) - 5- or 6-membered heterocycloalkyl($C_{1-4}$ alkyl),

(2) -

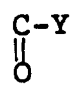

in which Y represents:

(a) - $C_{1-4}$ alkoxy,

(b) - N,N'-(5- or 6-membered cycloalkyl) (ureido)

(c) - amino cyclizable at the R'-position.

**2.** The compounds characterized according to claim 1, in that they are:

- ethyl 4-methyl-3-phenyl-2-pyrrolecarboxylate,
- methyl 4-methyl-3-(3'-nitrophenyl)-2-pyrrolecarboxylate,
- 3-methylpyrrolo[2,3-c]quinolin-4(5H)-one,
- ethyl 3-[2'-(N-benzyloxycarbonylglycylamido)phenyl]-4-methyl-2-pyrrolecarboxylate,
- 1,4-dimethyl-3-(2'-nitrophenyl)pyrrole-2-(N,N'-cyclohexylureidocarboxamide) [sic],
- 5-butyl-1,4-dimethyl-2-(1,4-dioxa-2-ethylcarbaldehyde)-3-[2'-(N-benzyloxycarbonyl-N-methylamino)-phenyl]pyrrole [sic],
- 5-butyl-1,4-dimethyl-2-(1,4-dioxa-2-ethylcarbaldehyde)-3-[2'-(N-methylamino)phenyl]pyrrole [sic].

**3.** Process for preparing the compounds represented by the general formula (I) according to claims 1 and 2, characterized in that a nitrostyrene of formula II

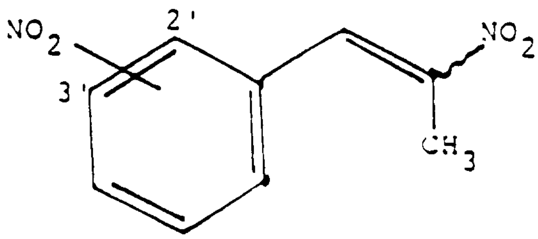

(II)

in which:
the $NO_2$ group is situated at the 2'- or 3'-position, is reacted with an alkyl isocyanoacetate of formula III

R-O-CO-$CH_2$-NC (III)

in which:
R is an ethyl or methyl group,
and, in the case where the compound I' is obtained, in that it is converted to other compounds represented by the general formula I

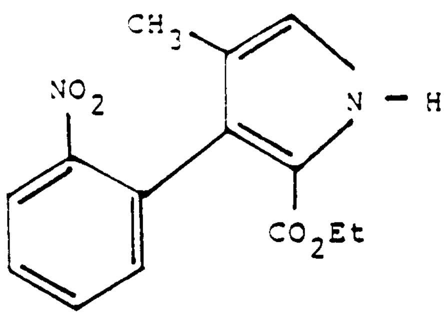

I'

by carrying out at least one of the following operations:

(1) - simultaneous reduction of the nitro group of the aromatic ring and the ester function of the pyrrole ring, and in this case cyclization,
(2) - reduction of the nitro group to amine,
(3) - acylation of the amine function obtained according to (2),
(4) - alkylation of the carbamate obtained according to (3), optionally followed by a hydrogenolysis,
(5) - saponification of the ester function of the pyrrole ring,
(6) - condensation of N,N'-cyclohexylurea [sic] with the acid function obtained according to (5),
(7) - reduction of the acid function obtained according to (5) to aldehyde,
(8) - Horner-Emmons condensation on the aldehyde function obtained according to (6),
(9) - electrophilic substitution on the pyrrole ring, and
(10) - intramolecular type cyclization reaction between the two aromatic rings.

**4.** The compounds represented by the general formula I according to claims 1 and 2, as drugs.

**5.** Pharmaceutical compositions, characterized in that they contain as active principle at least one compound of general formula I according to one of claims 1 and 2.

## Patentansprüche

**1.** Verbindungen, dadurch gekennzeichnet, daß sie durch die allgemeine Formel (I) dargestellt werden:

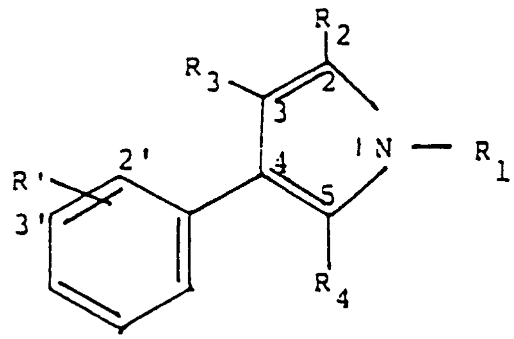

(I)

worin bedeuten:

$R_1$, $R_2$ und $R_3$ unabhängig voneinander
- (1) -H
- (2) $-C_{1-6}$-Alkyl,

R' das in 2'- oder 3'-Position vorliegt,
- (1) -H
- (2) $-NO_2$
- (3)

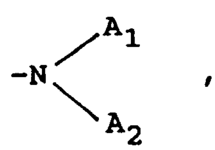

,

worin $A_1$ und $A_2$ unabhängig voneinander darstellen
- (a) -H
- (b) $-C_{1-4}$-Alkyl
- (c) $-C_{1-6}$-Alkylen oder $C_{1-6}$-Alkenylen, das an dem C-Atom in 5-Position des Pyrrol-Ringes einen Ring bildet (cyclisiert),
- (d)

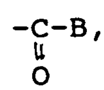

worin B steht für
- $C_{1-4}$-Alkoxyphenyl,
- N-Benzyloxycarbonylamino-$C_{1-4}$-Alkyl,
- $C_{1-6}$-Alkylen oder $C_{1-6}$-Alkenylen, das an dem C-Atom in 5-Position des Pyrrol-Ringes einen Ring bildet (cyclisiert),

$R_4$
- (1) 5- oder 6-gliedriges Heterocycloalkyl-($C_{1-4}$-alkyl),
- (2)

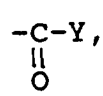

worin Y steht für
- (a) - $C_{1-4}$-Alkoxy,

(b) - 5- oder 6-gliedriges N,N'-Cycloalkyl(ureido),
(c) in R'-Position cyclisierbares Amino.

**2.** Verbindungen nach Anspruch 1, dadurch gekennzeichnet, daß es sich dabei handelt um:
- 4-Methyl-3-phenyl-2-pyrrol-ethylcarboxylat
- 4-Methyl-3-(3'-nitro-phenyl)-2-pyrrol-methylcarboxylat
- 3-Methyl-[2,3-c]pyrrolo-4(5H)-chinolin-on,
- 3-(2'-N-Benzyloxycarbonylglycylamido-phenyl)-4-methyl-2-pyrrol-ethylcarboxylat,
- 1,4-Dimethyl-3-(2'-nitro-phenyl)-2-pyrrol-N,N'-cyclohexylureidocarboxamid,
- 5-Butyl-1,4-dimethyl-2-(1,4-dioxa-2-ethylcarbaldehyd)-3-(N-benzyloxycarbonyl-2'-N-methylamino-phenyl)pyrrol,
- 5-Butyl-1,4-dimethyl-2-(1,4-dioxa-2-ethylcarbaldehyd)-3-(2'-N-methylamino-phenyl)pyrrol.

**3.** Verfahren zur Herstellung der Verbindungen der allgemeinen Formel (I) nach den Ansprüchen 1 und 2, dadurch gekennzeichnet, daß man ein Nitrostyrol der Formel (II)

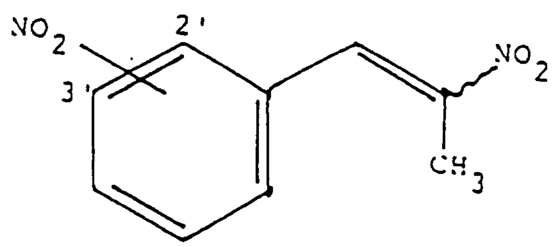

(II)

worin die $NO_2$-Gruppe in 2'- oder 3'-Position vorliegt,
mit einem Isocyanatoalkylacetat der Formel (III) reagieren läßt

$R-O-CO-CH_2-NC$ (III)

worin R für eine Ethyl- oder Methylgruppe steht, und,
für den Fall, daß die Verbindung (I') erhalten wird,

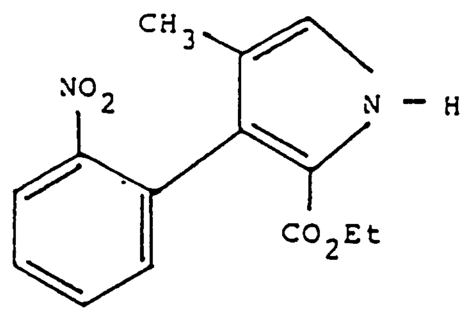

I'

diese in andere Verbindungen der allgemeinen Formel (I) umwandelt, indem man mindestens eine der folgenden Operationen durchführt:

(1) - gleichzeitige Reduktion der Nitrogruppe des aromatischen Ringes und der Esterfunktion des Pyrrolringes und in diesem Falle Cyclisierung,
(2) - Reduktion der Nitrogruppe zu einer Amingruppe,
(3) - Acylierung der gemäß (2) erhaltenen Aminfunktion,
(4) - Alkylierung des gemäß (3) erhaltenen Carbamats und gegebenenfalls anschließende hydrierende Spaltung,
(5) - Verseifung der Esterfunktion des Pyrrolringes,
(6) - Kondensation des N,N'-Cyclohexylharnstoffs an der gemäß (5) erhaltenen Säurefunktion,
(7) - Reduktion der gemäß (5) erhaltenen Säurefunktion zu einer Aldehydfunktion,
(8) - Horner-Emmons-Kondensation an der gemäß (6) erhaltenen Aldehydfunktion,

(9) - elektrophile Substitution an dem Pyrrolring und
(10) - Cyclisierungsreaktion vom intramolekularen Typ zwischen den beiden aromatischen Kernen (Ringen).

**4.** Verbindungen, dargestellt durch die allgemeine Formel (I) nach den Ansprüchen 1 und 2, als Arzneimittel.

**5.** Pharmazeutische Zusammensetzungen, dadurch gekennzeichnet, daß sie als aktives Prinzip (Wirkstoff) mindestens eine Verbindung der allgemeinen Formel (I) nach einem der Ansprüche 1 und 2 enthalten.